# EUROPÄISCHE PATENTANMELDUNG

(11) **EP 1 889 602 A2**
(43) Veröffentlichungstag der Anmeldung: **20.02.2008**
(21) Anmeldenummer: 07110558.9
(22) Anmeldetag: 19.06.2007
(51) Int. Cl.: A61K 8/42, A61K 8/49, A61K 8/73, A61K 8/81, A61Q 5/02, A61Q 5/06, A61Q 5/12, A61Q 19/00, A61Q 19/10

(54) **Kosmetische Zubereitungen zur Restrukturierung von Haaren mit besonderen Harnstoffderivaten**

(30) Priorität: 24.07.2006 DE 102006034533
(71) Anmelder: Beiersdorf AG, 20253 Hamburg (DE)
(72) Erfinder: Schmidt-Rose, Thomas, 20253, Hamburg (DE); Cerv, Svenja, 22761, Hamburg (DE); Wöhrmann, Michael, 22525, Hamburg (DE); Lehmbeck, Frank, 21033, Hamburg (DE)

(57) **Zusammenfassung**

Kosmetische Zubereitung zur Restrukturierung von Haaren enthaltend
a) Ein hydroxyalkyliertes Harnstoffderivat und
b) mindestens eine kationische Pflegekomponente, bevorzugt kationische Tenside und/oder kationische Polymere.

## Beschreibung

Die Erfindung betrifft kosmetische Zubereitungen, insbesondere Duschzubereitungen, Haarreinigungszubereitungen oder Haarpflegemittel, enthaltend besondere Harnstoffderivate. Die Zubereitungen sind mild zu Haut und Haaren und führen zu einer Verbesserung der Haarstruktur als auch der physikalisch-optischen Haareigenschaften.

Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt es eine soziale Funktion, da es über sein Erscheinungsbild erheblich zu zwischenmenschlichen Beziehungen und zum Selbstwertgefühl des Individuums beiträgt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft seinerseits ist aus drei Schichten aufgebaut: einem zentralen Teil - dem so genannten Haarmark (Medulla), welches allerdings beim Menschen zurückgebildet ist und oft gänzlich fehlt - ferner dem Mark (Cortex) und der äußeren, bis zu zehn Lagen starken Schuppenschicht (Cuticula), die das ganze Haar umhüllt.

Das menschliche Haar ist, sofern keine krankhaften Veränderungen vorliegen, in seinem frisch nachgewachsenen Zustand praktisch nicht zu verbessern. Der in der Nähe der Kopfhaut befindliche Teil eines Haares weist dementsprechend eine nahezu geschlossene Schuppenschicht auf. Insbesondere die Schuppenschicht als Außenhülle des Haares, aber auch der innere Bereich unterhalb der Cuticula sind besonderer Beanspruchung durch Umwelteinflüsse ausgesetzt.

Wesentliche Einflüsse für den Qualitätsverlust eines Haares während seiner Alterung sind der Einfluss des Sonnenlichts, mechanische Belastungen durch intensives Kämmen oder Bürsten, aber auch Haarbehandlungen, wie Haarfärbungen und insbesondere Blondierungen sowie Haarverformungen, beispielsweise Dauerwellverfahren. Besonders oxidative Belastungen führen demnach häufig zu einer Schädigung des Haares.

Sowohl UV-A- als auch UV-B-Strahlung haben einen schädigenden Einfluss auf das Haar, der sich beispielsweise darin äußert, dass bestimmte Aminosäuren wie Cystin und Methionin abgebaut oder Schwefel-Schwefel-Bindungen des Keratins gespalten werden, was im schlimmsten Fall eine Zerstörung des Haars zur Folge haben kann. Weiterhin stellen Haar und Kopfhaut Teile des Körpers dar, die aufgrund ihrer Position beim Aufenthalt im Freien einer erheblichen Menge an UV-Strahlung ausgesetzt sind.

Ein Ziel der Haarpflege und damit auch der vorliegenden Erfindung ist es, den Naturzustand des frisch nachgewachsenen Haares über einen möglichst langen Zeitraum zu erhalten und im Fall eines Verlusts wieder herzustellen. Seidiger Glanz, geringe Porosität und ein angenehmes, glattes Gefühl gelten als Merkmale für natürliches, gesundes Haar.

Seit Ende des vergangenen Jahrhunderts werden Produkte zur Haarpflege gezielt entwickelt. Dies führte zu einer Vielzahl von Präparaten sowohl für die allgemeine Haarpflege als auch zur Behebung von Anomalien des Haares und der Kopfhaut. Im allgemeinen werden heutzutage Haarpflegekosmetika verwendet, welche entweder dazu bestimmt sind, nach dem Einwirken aus dem Haar wieder ausgespült zu werden, oder welche auf dem Haar verbleiben sollen. Letztere können so formuliert werden, dass sie nicht nur der Pflege des einzelnen Haars dienen, sondern auch das Aussehen einer Frisur insgesamt verbessern. Solcherart gepflegtes Haar zeichnet sich durch einen angenehmen Griff, natürlichen Glanz, vermehrte Fülle, Geschmeidigkeit und somit gute Frisierbarkeit und Festigkeit und somit gutem Frisurensitz aus.

Produkte die ausschließlich der Pflege des Haares dienen, werden allgemein als Haarkonditioniermittel oder Conditioner bezeichnet. Diese können nach einer mehr oder weniger langen Verweilzeit auf dem Haar ausgespült werden (Rinse-off Produkte, z. B. Spülungen, Haar-Kuren) oder sie verbleiben nach der Anwendung auf dem Haar (Leave-on Produkte). Die Produkte können verschiedene Konsistenzen haben, so dass sie ganz unterschiedlich appliziert werden können. Es können Emulsionen oder Gele sein oder dünnflüssige Lösungen, die z. B. über Sprühapplikationen aufgebracht werden, oder Schäume, die z. B. durch geeignete Druckgaspackungen oder spezielle Schaumpumpen bei der Applikation erzeugt werden. Cremige, trübe und klar transparente Produkte sind im Markt zu finden.

Je nach Verwendungszweck findet man ganz unterschiedliche Wirkstoffe oder Kombinationen von Wirkstoffen in solchen Conditionern. Manche, die eher dem Schutz des Haares dienen, wie Antioxidantien oder UV-Filter, andere die das Haar geschmeidig machen wie z. B. kationische Tenside. Eine immer größere Bedeutung bekommen polymere Wirkstoffe, die je nach Art, Molgewicht und Ladung ganz unterschiedliche Eigenschaften haben. Im Vordergrund steht dabei jedoch eindeutig eine Verbesserung der Oberflächenbeschaffenheit des Haares.

Trotz einer großen Vielfalt von Produkten, die dem Verbraucher zur Verfügung stehen, sind einige Nachteile noch nicht restlos beseitigt. So zeigen Polymere, die dem Haar eher eine gewisse Festigkeit und somit Volumen geben häufig ein schlechtes Griffempfinden und eine schlechte Kämmbarkeit; Polymere, die das Haar geschmeidig machen führen häufig zu einer Beschwerung, was mit mangelndem Volumen verbunden ist. Auch der Einsatz von Stärkederivaten konnte in der Vergangenheit den geschilderten Nachteilen des Standes der Technik nicht abhelfen.

Der Stand der Technik lässt es an Haarpflegeformulierungen mangeln, welche dem geschädigten Haar in befriedigender Weise Pflege zukommen ließen. Aufgabe ist es daher, auch diesen Nachteilen des Standes der Technik Abhilfe zu schaffen.

Zwar kennt der Fachmann Zubereitungen mit Harnstoffderivaten. So offenbart beispielsweise die WO 2006/018198 sowie die deutschen Offenlegungsschriften, welche die Priorität dieser internationalen Anmeldung begründen, derartige Zubereitungen, die sich in ihrer Zusammensetzung jedoch deutlich von den erfindungsgemäßen Zubereitungen unterscheiden. Diese Schriften konnten nicht den Weg zur vorliegenden Erfindung weisen.

Ziel der vorliegenden Erfindung ist es auch Formulierungsvarianten zu schaffen, die verbesserte sensorische und taktile Eigenschaften aufweisen als Zubereitungen des Standes der Technik.

Die Aufgabe der vorliegenden Erfindung ist auch eine kosmetische Reinigungs- und Pflegezubereitung bereit zu stellen, die sich insbesondere durch gute Haut- und Haarverträglichkeit, gutes Schaumverhalten und angenehme Haarpflege und -frisieren auszeichnen.

Gelöst wird dieses Bündel an Aufgaben durch eine kosmetische Zubereitung zur Restrukturierung von Haaren enthaltend ein hydroxyalkyliertes Harnstoffderivat und mindestens eine kationische Pflegekomponente, bevorzugt kationische Tenside und/oder kationische Polymere. Besonders geeignete hydroxyalkylierte Harnstoffderivate sind 2-Hydroxyethylharnstoff, N-(2-Hydroxypropyl)harnstoff, N-(3-Hydroxypropyl)harnstoff, N-(2-Dimethylaminopropyl)harnstoff, N-(3-Dimethylaminopropyl)harnstoff, 1-(3-Hydroxyphenyl)harnstoff, 1-(2-Hydroxyethyl)-2-imidazolidinon, 1-(2-Hydroxypropyl)-2-imidazolidinon, 1-(3-Hydroxypropyl)-2-imidazolidinon, 4,5-Dihydroxy-1,3-dimethyl-imidazolidin-2-on, 1,3-bis(2-Hydroxyethyl)-2-imidazolidinon. Vorteilhaft ist es, wenn das hydroxyalkylierte Harnstoffderivat Hydroxyalkylharnstoff, bevorzugt n-Hydroxyalkylharnstoff, besonders bevorzugt 2-Hydroxyäthylharnstoff und/oder 1-Hydroxyalkyl-2-imidazolidinone, bevorzugt 1-(n-Hydroxyalkyl)-2-imidazolidinone, besonders bevorzugt 1-(2-Hydroxyethyl)-2-imidazolidinon ist.

Weiter ist es bevorzugt, wenn der Gehalt an hydroxyalkyliertem Harnstoffderivat 0,001 bis 5 Gew.-% beträgt, besonders bevorzugt 0,01 bis 2 Gew.-%, ganz besonders bevorzugt 0,05 bis 1,5 Gew.-%. Dabei ist es bevorzugt, wenn als kationisches Tensid 1 bis 10 Gew.-% quaternäre Ammoniumsalze enthalten sind. Dabei ist es bevorzugt, wenn als kationisches Polymer 0,1 bis 5 Gew.-% kationische Polymere, besonders bevorzugt PVP/VA Copolymere enthalten sind. Besonders bevorzugt sind kationische Polymere und kationische Tenside enthalten. Weiterhin ist es besonders bevorzugt, wenn ein Fettalkohol von 0,1 bis 10 Gew.-% enthalten ist. Besonders bevorzugt ist es, wenn ein weiteres Tensid aus der Gruppe der anionischen, amphoteren oder nicht ionischen Tenside in Konzentrationen von 0,1 bis 20 Gew.-%, bevorzugt 3 bis 10 Gew.-%, besonders bevorzugt 4 bis 7 Gew.-% enthalten ist. Gleichfalls ist es bevorzugt, wenn die Zubereitung eine Emulsion mit weniger als 10 Gew. % Emulgator darstellt. Die Erfindung umfasst auch die Verwendung einer solchen Zubereitung zur topischen Anwendung auf Haut und/oder Haaren, die Verwendung einer solchen Zubereitung als Duschzubereitung, Haarpflegezubereitung, Shampoo oder Haarpflegemittel zur Anwendung an Mensch oder Tier sowie die Verwendung einer solchen Zubereitung zum Schutz des Haares vor Abrieb, vor dem Ausbleichen und/oder zur Verbesserung der Kämmbarkeit des Haares.

Die Erfindung umfasst darüber hinaus auch die Verwendung der Zubereitungen auf der Haut und/oder dem Haar.

Bei Haarpflegeformulierungen ist darüber hinaus ein positiver Einfluss auf die Sensorik zu spüren, der sich in einem angenehmeren Griff äußert.

Durch die topische Anwendung der Zubereitung auf der Haut und/oder dem Haar werden insbesondere folgende positive Eigenschaften des behandelten Haares bzw. Haut festgestellt:
• Verbesserung der Frisierbarkeit des Haares
• Vermeidung des negativ Effektes der statischen Aufladung der Haare
• stabilisierenden Effekt auf die Haarstruktur
• Schutz und Pflege der Haut
• pflegend für Haar und Kopfhaut, insbesondere über einen längeren Zeitraum nach der Anwendung
• strukturglättende Wirkung auf Haut und Haar
• Verleiht dem Haar natürlichen Schimmer und attraktives Volumen
• das Haar ist leichter zu kämmen und einfacher zu stylen
• schützt das Haar und die Kopfhaut beim Fönen
• schützt vor elektrostatischer Aufladung durch Balancierung und Ausgleich des Feuchtigkeitshaushaltes der Haare

Bevorzugt werden die erfindungsgemäßem Zubereitungen silikonfrei hergestellt und vertrieben.

Auch als Konditioner zeigten sich überraschend positive Eigenschaften, so wirkt das mit den erfindungsgemäßen Zubereitungen behandelte Haar äußerst gepflegt und lässt sich gut frisieren. Dies ist beispielsweise an der Kämmbarkeit, des Anscheins und der Fülle des Haares erkennbar.

Die erfindungsgemäßen Zubereitungen bzw. Verwendungen können in Form von Cremes, Gelen oder Lotionen vorliegen. So können sie z. B. eine Lösung, eine Emulsion vom Typ Wasser-in-Öl (W/O) oder vom Typ Öl-in-Wasser (O/W), oder eine multiple Emulsion, beispielsweise vom Typ Wasser-in-Öl-in-Wasser (W/O/W), Öl-in-Wasser-in-Öl (O/W/O), ein Gel, eine Hydrodispersion, eine lamellare Phase, eine flüssige isotrope Lösungsphase, eine micellare Phase, eine solide oder dispergierte ein- oder mehrfach hexagonale Phase, eine solide oder dispergierte ein- oder mehrfach kubische Phase, eine lyotrope Phase, eine kristalline Phase, einen festen Stift oder auch ein Aerosol darstellen.

Weiterhin können die erfindungsgemäßen Zubereitungen bzw. Verwendungen vorteilhaft in Form von Haarwässern, Tinkturen, Reinigungsformulierungen sowie in Form von Tonics oder Shampoos vorliegen.

Vorteilhaft wird der pH-Wert der erfindungsgemäßen Zubereitungen bzw. Verwendungen im schwach sauren bis neutralen Bereich eingestellt, bevorzugt von 3,0 - 8,0 , besonders bevorzugt von 5,0 - 6,5.

Es ist dem Fachmann natürlich bekannt, dass kosmetische Zubereitungen zumeist nicht ohne die üblichen Hilfs- und Zusatzstoffe denkbar sind. Die erfindungsgemäßen kosmetischen und dermatologischen Zubereitungen können dementsprechend ferner kosmetische Hilfsstoffe enthalten, wie sie üblicherweise in solchen Zubereitungen verwendet werden; beispielsweise Konsistenzgeber, Konservierungsmittel, Stabilisatoren, Füllstoffe, Parfüme, Pigmente, die färbende Wirkung haben, Verdickungsmittel, Suspendiermittel, Puffergemische, oberflächenaktive Substanzen, Emulgatoren, weichmachende, anfeuchtende und/oder feuchthaltende Substanzen, entzündungshemmende Substanzen, zusätzliche Wirkstoffe wie Vitamine oder Proteine, Lichtschutzmittel, Insektenrepellentien, Bakterizide, Wasser, Salze, antimikrobiell, proteolytische oder keratolytisch wirksame Substanzen, Medikamente oder andere übliche Bestandteile einer kosmetischen oder dermatologischen Formulierung wie Alkohole, Polyole, Polymere, Schaumstabilisatoren, organische Lösungsmittel oder auch Elektrolyte.

Die jeweils einzusetzenden Mengen an Trägerstoffen können in Abhängigkeit von der Art des jeweiligen Produktes vom Fachmann durch einfaches Ausprobieren leicht ermittelt werden.

Vorteilhafte Konservierungsmittel im Sinne der vorliegenden Erfindung sind beispielsweise Benzylalkohol, Sorbinsäure und dessen Salze, Formaldehydabspalter (wie z. B. Diazolidinylharnstoff (Handelsbezeichnung Germall II von ISP), Imidazolidinylharnstoff (Handelsbezeichnung Germall 115) oder DMDM Hydantoin, welches beispielsweise unter der Handelsbezeichnung Glydant^{™} von der Fa. Lonza erhältlich ist), Methylisothiazolinon und entsprechende Derivate (Handelsname Kathon CG), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl-, Isopropyl-, und/oder Butylparaben), Phenoxyethanol, Ethanol, Triclosan, Benzoesäure und dergleichen mehr. Üblicherweise umfaßt das Konservierungssystem erfindungsgemäß ferner vorteilhaft auch Substanzen, die die Wirksamkeit von klassischen Konservierungsmitteln verbessern, wie beispielsweise Hexandiol, Pentandiol, Butylenglycol oder auch Methylpropandiol.

Es ist ebenfalls vorteilhaft, den Zubereitungen im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden, insbesondere bevorzugt sind alpha-Glycosylrutin (AGR) und/oder Taxifolin.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1-10 Gew.-%, bezogen auf das Gesamtgewicht der Zubereitung.

Es ist auch möglich und vorteilhaft im Sinne der vorliegenden Erfindung, einen Gehalt an UV-Schutzsubstanzen zu verwenden. Vorteilhaft können daher erfindungsgemäße Zubereitungen Substanzen enthalten, die UV-Strahlung im UVB-Bereich absorbieren, wobei die Gesamtmenge der Filtersubstanzen vozugsweise zwischen 0,1 Gew.-% bis 30 Gew.-% betragen kann. Bevorzugt sind handelsübliche wasser- oder öllösliche UVB-Filter. Es kann auch von Vorteil sein, in erfindungsgemäßen Zubereitungen UVA-Filter einzusetzen, die üblicherweise in kosmetischen und/oder dermatologischen Zubereitungen enthalten sind. Es können die gleichen Mengen eingesetzt werden, welche für UVB-Filter genannt wurden.

Die erfindungsgemäßen Wirkstoffe können auch besonders vorteilhaft in Mikroemulsionen und/oder Nanoemulsionen, beispielsweise wie in der deutschen Offenlegungsschrift DE-195 9 079 beschrieben, verwendet werden.

Vorteilhafte waschaktive kationische Tenside im Sinne der vorliegenden Erfindung sind quaternäre Tenside. Quaternäre Tenside enthalten mindestens ein N-Atom, das mit 4 Alkyl- oder Arylgruppen kovalent verbunden ist. Vorteilhaft sind beispielsweise Alkylbetain, Alkylamidopropylbetain und Alkylamidopropylhydroxysultain.

Vorteilhafte waschaktive amphotere Tenside im Sinne der vorliegenden Erfindung sind
**•** Acyl-/dialkylethylendiamine, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,

### Vorteilhafte waschaktive nicht-ionische Tenside im Sinne der vorliegenden Erfindung sind

• Alkanolamide, wie Cocamide MEA/ DEA/ MIPA,
• Ester, die durch Veresterung von Carbonsäuren mit Ethylenoxid, Glycerin, Sorbitan oder anderen Alkoholen entstehen,
• Ether, beispielsweise ethoxylierte Alkohole, ethoxyliertes Lanolin, ethoxylierte Polysiloxane, propoxylierte POE Ether und Alkylpolyglycoside wie Laurylglucosid, Decylglycosid und Cocoglycosid.

### Weitere vorteilhafte anionische Tenside sind

• Taurate, beispielsweise Natriumlauroyltaurat und Natriummethylcocoyltaurat,
• Ether-Carbonsäuren, beispielsweise Natriumlaureth-13 Carboxylat und Natrium PEG-6 Cocamide Carboxylat, Natrium PEG-7-Olivenöl-Carboxylat
• Phosphorsäureester und Salze, wie beispielsweise DEA-Oleth-10 Phosphat und Dilaureth-4 Phosphat,
• Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat.

### Weitere vorteilhafte amphotere Tenside sind

• N-Alkylaminosäuren, beispielsweise Aminopropylalkylglutamid, Alkylaminopropionsäure, Natriumalkylimidodipropionat und Lauroamphocarboxyglycinat.

### Weitere vorteilhafte nicht-ionische Tenside sind Alkohole.

### Weitere geeignete anionische Tenside im Sinne der vorliegenden Erfindung sind ferner

• Acylglutamate wie Di-TEA-palmitoylaspartat und Natrium Caprylic/ Capric Glutamat,
• Acylpeptide, beispielsweise Palmitoyl hydrolysiertes Milchprotein, Natrium Cocoyl hydrolysiertes Soja Protein und Natrium-/ Kalium Cocoyl hydrolysiertes Kollagen
   sowie Carbonsäuren und Derivate, wie
• beispielsweise Laurinsäure, Aluminiumstearat, Magnesiumalkanolat und Zinkundecylenat,
• Ester-Carbonsäuren, beispielsweise Calciumstearoyllactylat, Laureth-6 Citrat und Natrium PEG-4 Lauramidcarboxylat,
• Alkylarylsulfonate.

### Weitere geeignete kationische Tenside im Sinne der vorliegenden Erfindung sind ferner

• Alkylamine,
• Alkylimidazole,
• ethoxylierte Amine,
   insbesondere deren Salze.

Weitere geeignete nicht-ionische Tenside im Sinne der vorliegenden Erfindung sind ferner Aminoxide, wie Cocoamidopropylaminoxid.

Es ist vorteilhaft das oder die erfindungsgemäßen waschaktiven Tenside aus der Gruppe der Tenside zu wählen, welche einen HLB-Wert von mehr als 25 haben, besonders vorteilhaft sind solche, welchen einen HLB-Wert von mehr als 35 haben.

Es ist erfindungsgemäß besonders bevorzugt, wenn als Tenside anionische, amphotere und/oder nichtionische Tenside eingesetzt werden wobei es ganz besonders bevorzugt ist, wenn als Tenside Natriumlaurylethersulfat, Cocoamidopropylbetain und/oder Natriumlauroylsulfosuccinat eingesetzt werden.

Eine erfindungsgemäß besonders vorteilhafte Ausführungsform der vorliegenden Erfindung stellt insbesondere die Kombination der Tenside von Natriumlaurylethersulfat mit Cocoamidopropylbetain dar.

Zur Anwendung werden die erfindungsgemäßen kosmetischen und/oder dermatologischen Formulierungen in der für Kosmetika und Dermatika üblichen Weise auf die Haut, Kopfhaut und/oder die Haare in ausreichender Menge aufgebracht.

Erfindungsgemäß vorteilhaft können zusätzlich sowohl anionische, kationische, nichtionische und zwitterionische Tenside eingesetzt werden.

Vorteilhafte waschaktive anionische Tenside im Sinne der vorliegenden Erfindung sind Acylaminosäuren und deren Salze, wie
• Acylglutamate, insbesondere Natriumacylglutamat
• Sarcosinate, beispielsweise Myristoyl Sarcosin, TEA-lauroyl Sarcosinat, Natriumlauroylsarcosinat und Natriumcocoylsarkosinat,

### Sulfonsäuren und deren Salze, wie

• Acylisethionate, z.B. Natrium-/ Ammoniumcocoylisethionat,
• Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat, Dinatrium PEG-5 Laurylcitratsulfosuccinat und Derivate,
   sowie Schwefelsäureester, wie
• Alkylethersulfat, beispielsweise Natrium-, Ammonium-, Magnesium-, MIPA-, TIPA- Laurethsulfat, Natriummyrethsulfat und Natrium C₁₂₋₁₃ Parethsulfat,
• Alkylsulfate, beispielsweise Natrium-, Ammonium- und TEA- Laurylsulfat.

Vorteilhafte waschaktive kationische Tenside im Sinne der vorliegenden Erfindung sind quaternäre Tenside. Quaternäre Tenside enthalten mindestens ein N-Atom, das mit 4 Alkyl- oder Arylgruppen kovalent verbunden ist. Vorteilhaft sind beispielsweise Alkylbetain, Alkylamidopropylbetain und Alkylamidopropylhydroxysultain.

### Vorteilhafte waschaktive amphotere Tenside im Sinne der vorliegenden Erfindung sind

• Acyl-/dialkylethylendiamine, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,

### Vorteilhafte waschaktive nicht-ionische Tenside im Sinne der vorliegenden Erfindung sind

• Alkanolamide, wie Cocamide MEA/ DEA/ MIPA,
• Ester, die durch Veresterung von Carbonsäuren mit Ethylenoxid, Glycerin, Sorbitan oder anderen Alkoholen entstehen,
• Ether, beispielsweise ethoxylierte Alkohole, ethoxyliertes Lanolin, ethoxylierte Polysiloxane, propoxylierte POE Ether und Alkylpolyglycoside wie Laurylglucosid, Decylglycosid und Cocoglycosid.

### Weitere vorteilhafte anionische Tenside sind

• Taurate, beispielsweise Natriumlauroyltaurat und Natriummethylcocoyltaurat,
• Ether-Carbonsäuren, beispielsweise Natriumlaureth-13 Carboxylat und Natrium PEG-6 Cocamide Carboxylat, Natrium PEG-7-Olivenöl-Carboxylat
• Phosphorsäureester und Salze, wie beispielsweise DEA-Oleth-10 Phosphat und Dilaureth-4 Phosphat,
• Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat.

### Weitere vorteilhafte amphotere Tenside sind

• N-Alkylaminosäuren, beispielsweise Aminopropylalkylglutamid, Alkylaminopropionsäure, Natriumalkylimidodipropionat und Lauroamphocarboxyglycinat und N-Kokosfettsäureamidoethyl-N-hydroxyethylglycinat Natriumsalze und deren Derivate.

### Weitere vorteilhafte nicht-ionische Tenside sind Alkohole.

### Weitere geeignete anionische Tenside im Sinne der vorliegenden Erfindung sind ferner

• Acylglutamate wie Di-TEA-palmitoylaspartat und Natrium Caprylic/ Capric Glutamat,
• Acylpeptide, beispielsweise Palmitoyl hydrolysiertes Milchprotein, Natrium Cocoyl hydrolysiertes Soja Protein und Natrium-/ Kalium Cocoyl hydrolysiertes Kollagen sowie Carbonsäuren und Derivate, wie
• beispielsweise Laurinsäure, Aluminiumstearat, Magnesiumalkanolat und Zinkundecylenat,
• Ester-Carbonsäuren, beispielsweise Calciumstearoyllactylat, Laureth-6 Citrat und Natrium PEG-4 Lauramidcarboxylat,
• Alkylarylsulfonate.

### Weitere geeignete kationische Tenside im Sinne der vorliegenden Erfindung sind ferner

• Alkylamine,
• Alkylimidazole,
• ethoxylierte Amine
   insbesondere deren Salze.

Weitere geeignete nicht-ionische Tenside im Sinne der vorliegenden Erfindung sind ferner Aminoxide, wie Cocoamidopropylaminoxid.

Es ist vorteilhaft das oder die erfindungsgemäßen waschaktiven Tenside aus der Gruppe der Tenside zu wählen, welche einen HLB-Wert von mehr als 25 haben, besonders vorteilhaft sind solche, welchen einen HLB-Wert von mehr als 35 haben.

Erfindungsgemäß bevorzugte anionische Tenside sind sind N-Acylaminosäuren, Alkylsulfate oder Alkylethersulfate bzw. eine Tensidkombinationen aus Alkylethersulfaten mit amphoteren oder nichtionischen Tensiden, wobei eine Tensidkombination aus aus Alkylethersulfaten mit Alkylamidopropylbetainen oder Alkylamphoacetaten oder Alkylpoyglucosiden besonders bevorzugt ist. Besonders bevorzugt werden auch Kombinationen aus Acylglutamaten und Alkylpolyglucosiden.

Es ist vorteilhaft im Sinn der vorliegenden Erfindung, wenn der Gehalt an einem oder mehreren waschaktiven Tensiden in der entsprechenden Teilzubereitung aus dem Bereich von 0,1 bis 30 Gewichts-%, bevorzugt von 0,1 bis 25 Gew.-%, ganz besonders vorteilhaft von 0,1 bis 10 Gew.-% gewählt wird, jeweils bezogen auf das Gesamtgewicht der Teilzubereitung.

Die folgenden Beispiele sollen die Verkörperungen der vorliegenden Erfindungen verdeutlichen. Allen Angaben sind in Gewichtsprozent angegeben.

### Rezepturbeispiel 1: Shampoo

| **Rohstoff (INCI)** | **1** | **2** | **3** | **4** | **5** | **6** |
|---|---|---|---|---|---|---|
| Cocamidopropyl Betaine | 10,00 | 10,00 | 10,00 | 10,00 | 10,00 | 10,00 |
| Sodium Laureth Sulfate | 36,00 | 36,00 | 36,00 | 36,00 | 36,00 | 36,00 |
| PEG-40 Hydrogenated Castor Oil | 0,60 | 0,60 | 0,60 | 0,60 | 0,60 | 0,60 |
| Polyquaternium-10 | - | - | - | - | 0,20 | 0,20 |
| Sodium Benzoate | 0,45 | 0,45 | 0,45 | 0,45 | 0,45 | 0,45 |
| Sodium Chloride | 2,00 | 2,00 | 2,00 | 2,00 | 2,00 | 2,00 |
| Citric Acid | 0,07 | 0,07 | 0,07 | 0,07 | 0,07 | 0,07 |
| Sodium Salicylate | 0,20 | 0,20 | 0,20 | 0,20 | 0,20 | 0,20 |
| (2-Hydroxyethyl)urea | - | 0,10 | - | - | 0,50 | - |
| 1-(2-Hydroxyethyl)-imidazolidin-2-on | - | - | 0,10 | - | - | 0,50 |
| 1,3,5-Tris(2-hydroxyethyl)cyanuric acid | - | - | | 0,50 | - | - |
| Parfum | 0,70 | 0,70 | 0,70 | 0,70 | 0,70 | 0,70 |
| Aqua | ad. 100 | ad. 100 | ad. 100 | ad. 100 | ad. 100 | ad. 100 |

### Rezepturbeispiel 2: Shampoo - Sebum-Balancing Shampoo

| **Rohstoff (INCI)** | **1** | **2** |
|---|---|---|
| Lauramidopropyl betaine | 7,50 | 7,50 |
| Sodium Laureth Sulfate | 25,00 | 25,00 |
| Cocamide DEA | 1,50 | 1,50 |
| Disodium laureth sulfosuccinate (and) sodium lauryl sulfoacetate | 5,00 | 5,00 |
| Sodium cocoyl wheat amino acids | 5,00 | 5,00 |
| Caprylyl/capryl glucoside | 2,50 | 2,50 |
| Disodium EDTA | 0,10 | 0,10 |
| Citric Acid | 0,42 | 0,42 |
| (2-Hydroxyethyl)urea | 0,50 | 0,50 |
| Garcinia Cambogia Extract (Hydroxycitrat) | 1,00 | 1,00 |
| Carnitin | 1,00 | 1,00 |
| (2-Hydroxyethyl)urea | 0,50 | - |
| 1-(2-Hydroxyethyl)-imidazolidin-2-on | - | 0,50 |
| Parfum, Preservatives | qs | qs |
| Aqua | Ad. 100 | Ad. 100 |

### Rezepturbeispiel 3: Haarwasser

| | **1** | **2** | **3** | **4** |
|---|---|---|---|---|
| Ethanol | 30,0 | 50,0 | - | - |
| Isopropanol | - | - | 40,0 | 30,0 |
| Panthenol | 0,2 | 0,1 | 0,2 | 0,2 |
| Menthol | 0,1 | - | 0,05 | 0,05 |
| Tocopheryl Acetate | 0,2 | 0,2 | - | 0,1 |
| C12-13 Alkyl Lactate | 0,2 | 0,1 | 0,2 | - |
| (2-Hydroxyethyl)urea | 0,50 | 0,25 | - | 0,50 |
| 1-(2-Hydroxyethyl)-imidazolidin-2-on | - | 0,25 | 0,50 | - |
| Parfüm, Konservierungsmittel | q.s. | q.s. | q.s. | q.s. |
| Wasser | ad 100 | ad 100 | ad 100 | ad 100 |

### Rezepturbeispiel 4: Haarstyling Gel

| | **1** | **2** | **3** |
|---|---|---|---|
| PVP/VA Copolymer | 5,0 | 6,0 | 7,0 |
| Carbomer | 0,5 | | 0,8 |
| Acrylatcopolymer (Acrylates/C 10-30 Alkyl Acrylate Crosspolymer) | - | 1,0 | - |
| PEG-40 hydriertes Rizinusöl | 0,2 | 0,2 | 0,2 |
| Silikonöl | - | - | 0,1 |
| Glycerin | 3,0 | - | - |
| NaOH | q.s. | q.s. | q.s. |
| Parfum | 0,3 | 0,3 | 0,3 |
| (2-Hydroxyethyl)urea | 1,00 | - | 1,00 |
| 1-(2-Hydroxyethyl)-imidazolidin-2-on | - | 1,00 | 1,00 |
| Ethanol | - | - | 10,0 |
| Wasser | ad 100 | ad 100 | ad 100 |

### Rezepturbeispiel 5: Schaumfestiger

| | Schaumfestiger starke Festigung | Schaumfestiger extra starke Festigung |
|---|---|---|
| Acrylates Copolymer (4) | 2,00 | 2,00 |
| Cocamidopropylbetain | 0,50 | 0,50 |
| Parfüm, Konservierungsmittel, pH-Einstellung, Lösungsvermittler | q.s. | q.s. |
| Propan/Butan | 8,00 | 8,00 |
| (2-Hydroxyethyl)urea | 0,75 | - |
| 1-(2-Hydroxyethyl)-imidazolidin-2-on | - | 0,75 |
| Wasser, VES | ad 100,00 | |

| | | |
|---|---|---|
| (4) anionisches Polymer z.B. von BASF: Luvimer MAEX | | |

### Rezepturbeispiel 6: Stylinggel

| | Stylinggel starke Festigung | Stylinggel extra starke Festigung |
|---|---|---|
| PVP/VA Copolymer | 2,00 | 4,00 |
| Acrylates Copolymer (4) | 2,00 | 2,00 |
| Carbomer | 0,50 | 0,50 |
| Parfüm, Konservierungsmittel, pH-Einstellung, Lösungsvermittler | q.s. | q.s. |
| Propylenglycol | 5,00 | 5,00 |
| (2-Hydroxyethyl)urea | - | 0,50 |
| 1-(2-Hydroxyethyl)-imidazolidin-2-on | 0,50 | - |
| Wasser, VES | ad 100,00 | |

| | | |
|---|---|---|
| (4) anionisches Polymer z.B. von BASF: Luvimer MAEX pH einstellen auf 6,0 | | |

### Rezepturbeispiel 7: Haarkur

| | **1** | **2** | **3** | **4** | **5** | **5** |
|---|---|---|---|---|---|---|
| Hydroxypropylmethylcellulose | 0,5% | 0,5% | 0,5% | 0,3% | 0,4% | 0,5% |
| Cetrimoniumbromid | 1,0 | - | 0,8 | - | 0,5 | 0,7 |
| Behentrimoniumchlorid | - | 0,7 | 0,3 | - | - | - |
| Distearoylethyl Hydroxyethylmonium Methosulfate | - | - | - | 1,2 | - | 0,7 |
| Palmitamidopropyltrimonium Chlorid | - | - | - | - | 0,5 | - |
| Glycerin | 3,0 | 3,0 | 3,0 | 2,5 | 2,0 | - |
| Cetearylalkohol | 2,5 | 2,5 | 2,5 | 2,5 | 2,0 | 2,5 |
| Glycerylstearat | 2,0 | 2,0 | 2,0 | 2,0 | 2,0 | 1,8 |
| Polyquaternium-10 | 0,1 | - | - | - | 0,1 | - |
| Guar Hydroxypropyl Trimonium Chlorid | - | 0,2 | - | 0,1 | - | - |
| (2-Hydroxyethyl)urea | 0,50 | - | 0,25 | 0,50 | - | 0,25 |
| 1-(2-Hydroxyethyl)-imidazolidin-2-on | - | 0,50 | 0,25 | - | 0,50 | 0,25 |
| Konservierungsmittel, Parfüm, Lösungsvermittler | q.s. | q.s. | q.s. | q.s. | q.s. | q.s. |
| Wasser | ad 100 | ad 100 | ad 100 | ad 100 | ad 100 | ad 100 |

### Rezepturbeispiel 8: Haarspülung

| | **1** | **2** | **3** | **4** | **5** | **6** |
|---|---|---|---|---|---|---|
| Cetrimoniumchlorid | 1,0 | 0,5 | 0,5 | - | 0,5 | 0,5 |
| Distearoylethyl Hydroxyethylmonium Methosulfate | - | - | - | 1,0 | - | 0,5 |
| Palmitamidopropyltrimonium Chlorid | - | - | - | - | 0,8 | - |
| Behentrimoniumchlorid | - | 0,2% | 0,3 | - | - | - |
| Cetearylalkohol | 3,0 | 2,5 | 2,8 | 3,0 | 2,6 | 2,8 |
| Glycerin | 3,0 | 3,0 | 3,0 | 2,8 | 2,5 | 2,0 |
| Hydroxyethylcellulose | 0,2 | 0,2 | 0,2 | 0,2 | 0,2 | 0,2 |
| Polyquaternium-10 | 0,1 | - | - | - | - | 0,1 |
| Guar Hydroxypropyl Trimonium Chlorid | - | 0,2 | - | - | - | - |
| (2-Hydroxyethyl)urea | 0,50 | - | 0,50 | - | 0,25 | 0,75 |
| 1-(2-Hydroxyethyl)-imidazolidin-2-on | - | 0,50 | - | 0,50 | 0,25 | - |
| Konservierungsmittel, Parfüm, Lösungsvermittler | q.s. | q.s. | q.s. | q.s. | q.s. | q.s. |
| Wasser | ad 100 | ad 100 | ad 100 | ad 100 | ad 100 | ad 100 |

### Rezepturbeispiel 9: Spray-Conditioner

| | **1** | **2** | **3** | **4** | **5** | **6** | **7** |
|---|---|---|---|---|---|---|---|
| Cetrimoniumchlorid | 0,2 | 0,1 | 0,8 | 0,5 | - | 0,2 | - |
| Behentrimoniumchlorid | - | 0,2 | 0,3 | - | - | - | - |
| Distearoylethyl Hydroxyethylmonium Methosulfate | - | - | - | - | 0,2 | - | 0,2 |
| Palmitamidopropyltrimonium Chlorid | - | - | - | - | - | 0,1 | 0,1 |
| Benzophenone-4 | 0,05 | 0,03 | - | - | - | - | 0,03 |
| PVP/VA Copolymer | - | 0,7 | - | - | - | 0,2 | - |
| Polyquaternium-10 | 0,1 | - | - | - | 0,1 | - | - |
| Polyquaternium-4 | 0,2 | - | - | - | - | 0,2 | 0,2 |
| Propylene Glycol | - | - | - | 3,0 | 2,0 | - | 2,0 |
| Polyquaternium-11 | - | - | 0,2 | - | - | - | - |
| Panthenol | 0,1 | - | 0,2 | 0,1 | - | 0,2 | - |
| Glyceryllsostearate | - | - | - | 0,4 | - | - | - |
| Isoceteth-20 | - | - | - | 0,8 | - | - | - |
| Dicaprylyl Carbonate | - | - | - | 0,5 | - | - | - |
| (2-Hydroxyethyl)urea | 0,75 | - | 0,25 | 0,50 | 0,25 | - | - |
| 1-(2-Hydroxyethyl)imidazolidin-2-on | - | 0,75 | 0,50 | - | - | 0,25 | 0,40 |
| Konservierungsmittel, Parfüm, Lösungsvermittler | q.s. | q.s. | q.s. | q.s. | q.s. | q.s. | q.s. |
| Wasser | ad 100 | ad 100 | ad 100 | ad 100 | ad 100 | ad 100 | ad 100 |

### Rezepturbeispiel 10: Leave-on Conditioner

| | **1** | **2** | **3** | **4** | **5** | **6** | **7** |
|---|---|---|---|---|---|---|---|
| Cetylalcohol | 1,5 | 1,8 | 2,0 | - | 1,0 | 1,7 | 2,0 |
| Cetrimoniumchlorid | 0,3 | 0,1 | 0,5 | 0,5 | - | 0,2 | - |
| Behentrimoniumchlorid | - | 0,2 | - | - | - | - | 0,2 |
| Distearoylethyl Hydroxyethylmonium Methosulfate | - | - | - | - | 0,5 | - | 0,2 |
| Palmitamidopropyltrimonium Chlorid | - | - | - | - | - | 0,2 | - |
| Benzophenone-4 | 0,05 | 0,03 | - | 0,1 | - | 0,1 | - |
| PVP/VA Copolymer | 0,4 | - | - | - | 0,3 | - | - |
| Polyquaternium-37 | - | - | - | 1,0 | - | - | - |
| Polyquaternium-4 | - | - | - | 0,2 | - | 0,1 | - |
| Polyquaternium-10 | - | - | 0,5 | - | 0,3 | - | 0,2 |
| Panthenol | 0,1 | - | 0,2 | 0,1 | - | - | 0,2 |
| Hydroxyethylcellulose | - | - | - | 0,3 | - | - | 0,2 |
| Acrylates/C10-30 Alkyl Acrylates Crosspolymer | 0,5 | 0,3 | 0,2 | - | - | - | - |
| C12-13 Alkyl Lactate | 2,0 | 1,0 | 1,5 | 1,0 | 1,5 | 1,0 | 1,0 |
| Laureth-4 | - | - | - | 0,5 | - | - | - |
| Aluminium Starch Octenylsuccinate | - | - | - | 1,0 | - | - | - |
| Dicaprylyl Carbonate | - | - | - | 1,0 | - | - | - |
| (2-Hydroxyethyl)urea | 0,50 | 0,25 | 0,75 | 0,30 | - | - | - |
| 1-(2-Hydroxyethyl)imidazolidin-2-on | - | - | - | 0,30 | 0,50 | 0,25 | 0,75 |
| Konservierungsmittel, Parfüm, Lösungsvermittler | q.s. | q.s. | q.s. | q.s. | q.s | q.s | q.s |
| Wasser | ad 100 | ad 100 | ad 100 | ad 100 | ad 100 | ad 100 | ad 100 |

### Rezepturbeispiel 11: Conditioner-Shampoo mit Perlglanz

| | **1** | **2** | **3** | **4** | **5** | **6** |
|---|---|---|---|---|---|---|
| Natrium Laurylethersulfat | 9 | 9 | 9 | 9 | 9 | 9 |
| Cocamidopropyl Betain | 4 | 3 | 4 | 3 | 4 | 3 |
| Dinatrium PEG-5 Laurylcitrat Sulfosuccinat | 3 | 3 | 3 | 2 | 3 | 4 |
| Verdicker | 0.1 | 0.1 | 0.1 | 0.2 | 0,3 | 0,4 |
| Polyquaternium-10 | 0.3 | 0.1 | 0.1 | 0.3 | - | 0,2 |
| Guar Hydroxypropyl-Trimonium Chlorid | - | - | 0.1 | 0.1 | 0.2 | 0,3 |
| Perlglanz | 1.5 | 3 | 4 | 2 | 2,5 | 0,75 |
| Trübungsmittel | - | - | - | - | 0.5 | 0,5 |
| Iminodibersteinsäure | 0.1 | 0.2 | 0.1 | 0.5 | 0.5 | 0,5 |
| PEG-40 hydriertes Rizinusöl | 0.2 | 0.3 | 0.4 | 0.5 | 0.2 | 0,3 |
| Natrium Salicylat | 0.2 | 0.2 | 0.4 | 0.4 | 0.4 | 0,2 |
| Natrium Benzoat | 0.4 | 0.4 | 0.4 | 0.4 | 0.4 | 0,4 |
| Natriumchlorid | 0,9 | 1,0 | 1,2 | 1,5 | - | 1,5 |
| (2-Hydroxyethyl)urea | 0,50 | - | 0,75 | - | 0,40 | 0,20 |
| 1-(2-Hydroxyethyl)imidazolidin-2-on | - | 0,5 | - | 0,75 | 0,40 | 0,20 |
| Zitronensäure | q.s. | q.s. | q.s. | q.s. | q.s. | q.s. |
| Parfüm | q.s. | q.s. | q.s. | q.s. | q.s. | q.s. |
| Wasser | ad 100 | ad 100 | ad 100 | ad 100 | ad 100 | ad 100 |

### Rezepturbeispiel 12: Klares Conditioning-Shampoo

| | **1** | **2** | **3** | **4** | **5** | **6** |
|---|---|---|---|---|---|---|
| Natrium Laurylethersulfat | 10 | 9 | 3.5 | 3.5 | 0.5 | 8 |
| Natrium Myrethsulfat | - | - | 3.5 | 3.5 | 3.0 | 2 |
| Cocamidopropyl Betain | 4 | 4.5 | 3 | - | - | 3 |
| Natrium Cocoamphoacetat | - | - | - | 2.5 | - | 3 |
| Dinatrium PEG-5 Laurylcitrat Sulfosuccinat | - | - | - | - | 2.5 | 3 |
| Decylglucosid | - | - | - | 2 | 4.5 | - |
| (2-Hydroxyethyl)urea | 0,10 | 0,20 | - | 0,50 | - | 0,75 |
| 1-(2-Hydroxyethyl)imidazolidin-2-on | - | 0,20 | 0,10 | 0,50 | 0,75 | - |
| Verdicker | 0.1 | 0.2 | 0.3 | 0.4 | 0.5 | - |
| Polyquaternium-10 | 0.1 | 0.1 | 0.05 | 0.25 | 0.2 | 0,3 |
| Guar Hydroxypropyl-Trimonium Chlorid | - | 0.1 | - | - | 0.2 | - |
| Hydrolysiertes Seidenprotein | - | 0,1 | 0,2 | 0,3 | 0.3 | - |
| Iminodibersteinsäure | 0.1 | 0.1 | 0.2 | - | - | 0,5 |
| PEG-40 hydriertes Rizinusöl | 0.1 | 0.2 | 0.3 | 0.1 | 0.2 | 0,4 |
| Natrium Salicylat | - | - | 0.4 | - | | 0,2 |
| Natrium Benzoat | 0.5 | 0.5 | 0.4 | 0.4 | 0.4 | 0,4 |
| Benzophenon-4 | - | - | 0.1 | 0,2 | 0,3 | 0,4 |
| Zitronensäure | q.s. | q.s. | q.s. | q.s. | q.s. | q.s. |
| Parfüm | q.s. | q.s. | q.s. | q.s. | q.s. | q.s. |
| Wasser | ad 100 | ad 100 | ad 100 | ad 100 | ad 100 | ad 100 |

### Rezepturbeispiel 13: Mildes Baby Shampoo)

| | **1** | **2** | **3** | **4** | **5** | **6** |
|---|---|---|---|---|---|---|
| Natrium Myristylethersulfat | 4 | 4 | 5 | 5 | 4 | 4 |
| Decylglucosid | 4 | 4 | 4 | 4 | 4 | 2 |
| Dinatrium PEG-5 Laurylcitrat Sulfosuccinat | 4 | 4 | 3 | 5 | 5 | 3 |
| PEG-80 Sorbitan Laurat | 2 | 1 | 1 | - | 0.5 | - |
| (2-Hydroxyethyl)urea | 1,00 | - | 0,75 | - | 0,50 | - |
| 1-(2-Hydroxyethyl)imidazolidin-2-on | - | 1,00 | - | 0,75 | - | 0,50 |
| Verdicker | 0.1 | 0.1 | 0.1 | 0.2 | 0.3 | 0,4 |
| Polyquaternium-10 | 0.3 | 0.1 | 0.1 | 0.3 | - | 0,1 |
| Guar Hydroxypropyl-Trimonium Chlorid | - | - | 0.1 | 0.2 | 0.2 | - |
| Perlglanz | - | - | 4 | 2 | 2.5 | 1 |
| Trübungsmittel | - | - | - | - | 0.5 | 0,5 |
| Iminodibersteinsäure | - | 0.2 | 0.1 | 0.5 | 0.5 | 0,5 |
| PEG-40 hydriertes Rizinusöl | 0.2 | 0.3 | 0.4 | 0.2 | 0.3 | 0,4 |
| Natrium Salicylat | 0.2 | 0.2 | 0.2 | 0.4 | 0.4 | 0,4 |
| Natrium Benzoat | 0.4 | 0.4 | 0.4 | 0.4 | 0.4 | 0,4 |
| Natriumchlorid | 0.9 | 1.0 | 1.2 | 1,5 | 1,8 | - |
| Zitronensäure | q.s. | q.s. | q.s. | q.s. | q.s. | q.s. |
| Parfüm | q.s. | q.s. | q.s. | q.s. | q.s. | q.s. |
| Wasser | ad 100 | ad 100 | ad 100 | ad 100 | ad 100 | ad 100 |

### Rezepturbeispiel 14: Antischuppen Shampoo/Mildes Kopfhaut Shampoo)

| | **1** | **2** | **3** | **4** | **5** | **6** |
|---|---|---|---|---|---|---|
| Natrium Laurylethersulfat | 9 | 9 | 9 | 9 | 10 | - |
| Natrium Myristylethersulfat | - | - | - | - | - | 6 |
| Cocamidopropyl Betain | 4 | 4 | 3 | 3 | 4 | 5 |
| Dinatrium PEG-5 Laurylcitrat Sulfosuccinat | 3 | 3 | 3 | 3 | - | - |
| (2-Hydroxyethyl)urea | 1,00 | - | 0,75 | - | 0,50 | - |
| 1-(2-Hydroxyethyl)imidazolidin-2-on | - | 1,00 | - | 0,75 | - | 0,50 |
| Natrium Cocoamphoacetat | - | - | - | - | - | 2.5 |
| Decylglucosid | - | - | - | - | - | 2.5 |
| Verdicker | 0.1 | 0.1 | 0.1 | 0.2 | 0.4 | 3 |
| Polyquaternium-10 | 0.3 | 0.1 | 0.1 | 0.3 | 0.1 | 0.3 |
| Guar Hydroxypropyl-Trimonium Chlorid | 0,1 | 0,1 | 0.2 | 0.2 | - | - |
| Climbazol | 0.5 | 0.5 | - | 0.5 | 1.0 | - |
| Piroctone Olamin | - | 0.5 | 0.3 | - | 0.5 | - |
| Laureth-9 | - | - | - | - | 2 | 2 |
| Panthenol | - | - | - | - | - | 0,1 |
| Harnstoff | | | | 3 | 4 | 5 |
| Perlglanz | 1.5 | 3 | 4 | 2 | 2.5 | - |
| Trübungsmittel | - | - | - | - | 0.5 | - |
| Iminodibersteinsäure | 0.1 | 0.2 | 0.1 | 0.5 | 0.5 | - |
| PEG-40 hydriertes Rizinusöl | 0.2 | 0.3 | 0.4 | 0.5 | 0.2 | - |
| Natrium Salicylat | 0.4 | 0.4 | 0.4 | 0.4 | 0.4 | 0.2 |
| Natrium Benzoat | 0.4 | 0.4 | 0.4 | 0.4 | 0.4 | 0.4 |
| Natriumchlorid | 0.9 | 1.0 | 1.2 | - | - | - |
| Zitronensäure | q.s. | q.s. | q.s. | q.s. | q.s. | - |
| Milchsäure | - | - | - | - | - | q.s. |
| Parfüm | q.s. | q.s. | q.s. | q.s. | q.s. | q.s |
| Wasser | ad 100 | ad 100 | ad 100 | ad 100 | ad 100 | ad 100 |

### Nachweis der restrukturierenden Wirkung an geschädigten Haaren:

- Verwendung von 10 cm langen und 1 cm breiten Naturhaartressen kaukasischer Herkunft (Fa. Kerling)
- Schädigung 20 min bei Raumtemperatur in 6% H₂O₂, anschließend 2 min Spülen unter fließendem Leitungswasser.
- Wirkstoffapplikation durch Einmassieren von 200µl Shampoo (2 min) mit anschließender Einwirkszeit von 8 min
- Abspülen für 2 Minuten unter fließendem Leitungswasser (37°C)
- Erzeugung des Kämmabriebs mittels automischer Nasskämmung (150x, Kämmmaschine Fa. SFC-Engineering)
- Auffangen des Kämmabriebs durch Ausspülen der Tressen nach jeweils 10 Kämmvorgängen in 15ml dest. H₂O
- photometrische Bestimmung des Gesamtproteingehaltes der Auffanglösung mittels kommerzieller Proteinbestimmungsmethode (BCA-Kit, Fa. Pierce)
- Doppelte Normierung des Kämmabriebs auf das Tressengewicht vor der Kämmung und auf Placebo

| Rezepturbeispiel | Proteinmenge | Signifikanz (n=6) |
|---|---|---|
| 1.1 (Placebo) | 100% | - |
| 1.2 | 56% | p = 0,0012 |
| 1.3 | 51% | p = 0,0045 |
| 1.4 | 66% | p = 0,0408 |

## Patentansprüche

1. Kosmetische Zubereitung zur Restrukturierung von Haaren enthaltend
a) Ein hydroxyalkyliertes Harnstoffderivat und
b) mindestens eine kationische Pflegekomponente, bevorzugt kationische Tenside und/oder kationische Polymere.

2. Zubereitung nach Anspruch 1 wobei das hydroxyalkylierte Harnstoffderivat Hydroxyalkylharnstoff, bevorzugt n-Hydroxyalkylharnstoff, besonders bevorzugt 2-Hydroxyäthylharnstoff und/oder 1-Hydroxyalkyl-2-imidazolidinone, bevorzugt 1-(n-Hydroxyalkyl)-2-imidazolidinone, besonders bevorzugt 1-(2-Hydroxyethyl)-2-imidazolidinon ist.

3. Zubereitung nach einem der vorangehenden Patentansprüche wobei der Gehalt an hydroxyalkyliertem Harnstoffderivat 0,001 bis 5 Gew.-% beträgt, bevorzugt 0,01 bis 2 Gew.-%, besonders bevorzugt 0,05 bis 1,5 Gew.-%.

4. Zubereitung nach einem der vorangehenden Patentansprüche **dadurch gekennzeichnet, dass** als kationisches Tensid 1 bis 10 Gew.-% quaternäre Ammoniusalze enthalten sind.

5. Zubereitung nach einem der vorangehenden Patentansprüche **dadurch gekennzeichnet, dass** als kationisches Polymer 0,1 bis 5 Gew.-% kationische Polymere, besonders bevorzugt PVP/VA Copolymere enthalten sind.

6. Zubereitung nach einem der vorangehenden Patentansprüche **dadurch gekennzeichnet, dass** ein weiteres Tensid aus der Gruppe der anionischen, amphoteren oder nicht ionischen Tenside in Konzentrationen von 0,1 bis 20 Gew.-%, bevorzugt 3 bis 10 Gew.-%, besonders bevorzugt 4 bis 7 Gew.-% enthalten ist.

7. Zubereitung nach einem der vorangehenden Patentansprüche **dadurch gekennzeichnet, dass** die Zubereitung eine Emulsion mit weniger als 10 Gew.-% Emulgator darstellt.

8. Verwendung der Zubereitung nach einem der vorangehenden Patentansprüche zur topischen Anwendung auf Haut und/oder Haaren.

9. Verwendung der Zubereitung nach einem der vorangehenden Patentansprüche als Duschzubereitung, Haarpflegezubereitung, Shampoo oder Haarpflegemittel zur Anwendung an Mensch oder Tier.

10. Verwendung der Zubereitung nach einem der vorangehenden Patentansprüche zum Schutz des Haares vor Abrieb, vor dem Ausbleichen und/oder zur Verbesserung der Kämmbarkeit des Haares.
